# EUROPÄISCHE PATENTSCHRIFT

(11) **EP 2 142 495 B1**
(45) Veröffentlichungstag und Bekanntmachung des Hinweises auf die Patenterteilung: **27.04.2016**
(21) Anmeldenummer: 08716566.8
(22) Anmeldetag: 15.03.2008
(51) Int. Cl.: C07C 65/28, A61K 31/194, A61P 9/00

(54) **SUBSTITUIERTE DIBENZOESÄURE-DERIVATE UND IHRE VERWENDUNG**
SUBSTITUTED DIBENZOIC ACID DERIVATIVES AND USE THEREOF
DERIVES D'ACIDE DIBENZOIQUE SUBSTITUES ET LEUR UTILISATION

(30) Priorität: 29.03.2007 DE 102007015035
(43) Veröffentlichungstag der Anmeldung: 13.01.2010
(73) Patentinhaber: Bayer Intellectual Property GmbH, 40789 Monheim (DE)
(72) Erfinder: HAHN, Michael, 40764 Langenfeld (DE); BECKER, Eva-Maria, 42327 Wuppertal (DE); KNORR, Andreas, 40699 Erkrath (DE); SCHNEIDER, Dirk, 42115 Wuppertal (DE); STASCH, Johannes-Peter, 42651 Solingen (DE); SCHLEMMER, Karl-Heinz, 42113 Wuppertal (DE); WUNDER, Frank, 42117 Wuppertal (DE); LANG, Dieter, 42553 Velbert (DE)
(74) Vertreter: BIP Patents
(86) Internationale Anmeldenummer: PCT/EP2008/002091
(87) Internationale Veröffentlichungsnummer: WO 2008/119458

(56) Entgegenhaltungen:
- WO-A-01/19355

## Beschreibung

Die vorliegende Anmeldung betrifft neue, substituierte Dibenzoesäure-Derivate, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prävention kardiovaskulärer Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-1-benzylindazol [YC-1, Wu et al., Blood 84 (1994), 4226; Mülsch et al., Br. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Br. J. Pharmacol. 114 (1995), 1587], sowie verschiedene substituierte Pyrazolderivate (WO 98/16223, WO 98/16507 und WO 98/23619).

Die vorstehend beschriebenen Stimulatoren der löslichen Guanylatcyclase stimulieren das Enzym entweder direkt über die Häm-Gruppe (Kohlenmonoxid, Stickstoffmonoxid oder Diphenyliodoniumhexafluorophosphat) durch Interaktion mit dem Eisenzentrum der Häm-Gruppe und eine sich daraus ergebende, zur Erhöhung der Enzymaktivität führende Konformationsänderung [Gerzer et al., FEBS Lett. 132 (1981), 71] oder über einen Häm-abhängigen Mechanismus, der unabhängig von NO ist, aber zu einer Potenzierung der stimulierenden Wirkung von NO oder CO führt [z.B. YC-1, Hoenicka et al., J. Mol. Med. 77 (1999) 14; oder die in WO 98/16223, WO 98/16507 und WO 98/23619 beschriebenen Pyrazolderivate].

Die in der Literatur behauptete stimulierende Wirkung von Isoliquiritigenin und von Fettsäuren, wie z.B. von Arachidonsäure, Prostaglandin-Endoperoxiden und Fettsäure-Hydroperoxiden auf die lösliche Guanylatcyclase konnte nicht bestätigt werden [vgl. z.B. Hoenicka et al., J. Mol. Med. 77 (1999), 14].

Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten bekannten Stimulatoren stimulierbar.

Es wurde eine Stimulation von Häm-freier löslicher Guanylatcyclase durch Protoporphyrin IX beschrieben [Ignarro et al., Adv. Pharmacol. 26 (1994), 35]. Allerdings kann Protoporphyrin IX als Mimik für das NO-Häm-Addukt angesehen werden, weshalb die Zugabe von Protoporphyrin IX zur löslichen Guanylatcyclase zur Bildung einer der durch NO stimulierten Häm-haltigen löslichen Guanylatcyclase entsprechenden Struktur des Enzyms führen dürfte. Dies wird auch durch die Tatsache belegt, dass die stimulierende Wirkung von Protoporphyrin IX durch den vorstehend beschriebenen NO-unabhängigen, aber Häm-abhängigen Stimulator YC-1 erhöht wird [Mülsch et al., Naunyn Schmiedebergs Arch. Pharmacol. 355, R47].

Im Gegensatz zu den vorstehend beschriebenen Stimulatoren der löslichen Guanylatcyclase sind die Verbindungen der vorliegenden Erfindung in der Lage, sowohl die Häm-haltige als auch die Häm-freie Form der löslichen Guanylatcyclase zu aktivieren. Die Stimulierung des Enzyms verläuft bei diesen neuen Aktivatoren also über einen Häm-unabhängigen Weg, was auch dadurch belegt wird, dass die neuen Aktivatoren am Häm-haltigen Enzym einerseits keine synergistische Wirkung mit NO zeigen und andererseits sich die Wirkung dieser neuartigen Aktivatoren nicht durch den Häm-abhängigen Inhibitor der löslichen Guanylatcyclase, 1*H*-1,2,4-Oxadiazol-(4,3-a)-chinoxalin-1-on (ODQ), blockieren lässt [Schmidt et al., J. Clin. Invest. 116 (2006), 2552; Stasch et al., Nature Rev. Drug Disc. 5 (2006), 755].

In EP 0 341 551-A1 werden Alkensäure-Derivate als Leukotrien-Antagonisten für die Behandlung von Erkrankungen des Kreislauf- und Atmungssystems offenbart. In WO 01/19355, WO 01/19776, WO 01/19778, WO 01/19780, WO 02/070462 und WO 02/070510 werden Dicarbonsäure- bzw. Aminodicarbonsäure-Derivate als Stimulatoren der löslichen Guanylatcyclase zur Behandlung von Herz-Kreislauf-Erkrankungen beschrieben. Allerdings zeigte es sich, dass diese Verbindungen hinsichtlich ihrer pharmakokinetischen Eigenschaften Nachteile aufweisen, wie insbesondere eine geringe Bioverfügbarkeit und/oder eine nur kurze Wirkdauer nach oraler Gabe.

Aufgabe der vorliegenden Erfindung war daher die Bereitstellung neuer Verbindungen, welche als Aktivatoren der löslichen Guanylatcyclase wirken, jedoch nicht die vorstehend aufgeführten Nachteile der Verbindungen aus dem Stand der Technik aufweisen.

Diese Aufgabe wird durch die in der vorliegenden Erfindung beschriebenen Verbindungen gelöst. Diese Verbindungen zeichnen sich strukturell im Vergleich zu den Verbindungen aus dem Stand der Technik durch eine 3-(Carboxyphenethyl)-5-phenylpent-4-en-1-yl-benzoesäure-Kernstruktur aus.

Im Einzelnen sind nicht erfindungsgemäße Verbindungen offenbart der allgemeinen Formel (I) 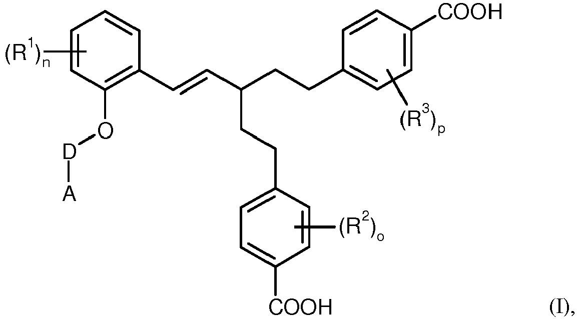 in welcher
- A: für eine Gruppe der Formel 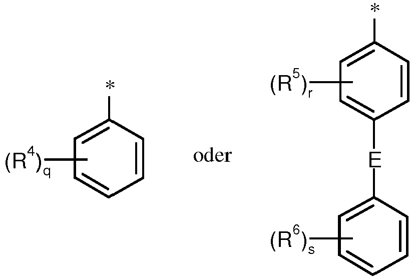
steht,
worin * die Verknüpfungsstelle mit der Gruppe D und
- E: eine Bindung, -CH₂-, -CH₂-CH₂- oder -CH=CH- bedeutet,
- D: für eine Bindung oder für (C₁-C₇)-Alkandiyl, (C₂-C₆)-Alkandiyl-O-^{#}, (C₂-C₇)-Alkendiyl oder (C₂-C₇)-Alkindiyl steht, welche jeweils ein- oder mehrfach mit Fluor substituiert sein können und worin ^{#} die Verknüpfungsstelle mit der Gruppe A bedeutet,

R¹, R², R³, R⁴, R⁵ und R⁶ unabhängig voneinander für einen Substituenten ausgewählt aus der Reihe Halogen, (C₁-C₆)-Alkyl, (C₁-C₆)-Alkoxy, Cyano und Nitro stehen, wobei Alkyl und Alkoxy ihrerseits ein- oder mehrfach mit Fluor substituiert sein können,
und
n, o, p, q, r und s unabhängig voneinander jeweils für die Zahl 0, 1, 2, 3 oder 4 stehen, wobei für den Fall, dass R¹, R², R³, R⁴, R⁵ oder R⁶ mehrfach auftreten, ihre Bedeutungen jeweils gleich oder verschieden sein können,
sowie ihre Salze, Solvate und Solvate der Salze.

Erfindungsgemäße Verbindungen sind die Verbindungen der Formel (I-A) gemäß Anspruch 1 und deren Salze, Solvate und Solvate der Salze, die von Formel (I-A) umfassten Verbindungen der nachfolgend genannten Formeln und deren Salze, Solvate und Solvate der Salze sowie die von Formel (I-A) umfassten, nachfolgend als Ausführungsbeispiele genannten Verbindungen und deren Salze, Solvate und Solvate der Salze, soweit es sich bei den von Formel (I-A) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, n-Propyl, Isopropyl, n-Butyl, *iso*-Butyl, *sec*.-Butyl, *tert.-Butyl,* 1-Ethylpropyl, n-Pentyl und n-Hexyl.
(C₁-C₇)-Alkandiyl und (C₂-C₆)-Alkandiyl stehen im Rahmen der Erfindung für einen geradkettigen oder verzweigten divalenten Alkylrest mit 1 bis 7 bzw. 2 bis 6 Kohlenstoffatomen. Bevorzugt ist ein geradkettiger Alkandiylrest mit 1 bis 6 bzw. 2 bis 5 Kohlenstoffatomen. Beispielhaft und vorzugsweise seien genannt: Methylen, 1,2-Ethylen, Ethan-1,1-diyl, 1,3-Propylen, Propan-1,1-diyl, Propan-1,2-diyl, Propan-2,2-diyl, 1,4-Butylen, Butan-1,2-diyl, Butan-1,3-diyl, Butan-2,3-diyl, Pentan-1,5-diyl, Pentan-2,4-diyl, 3-Methyl-pentan-2,4-diyl und Hexan-1,6-diyl.
(C₁-C₇)-Alkendiyl steht im Rahmen der Erfindung für einen geradkettigen oder verzweigten divalenten Alkenylrest mit 2 bis 7 Kohlenstoffatomen und bis zu 3 Doppelbindungen. Bevorzugt ist ein geradkettiger Alkendiylrest mit 2 bis 6 Kohlenstoffatomen und bis zu 2 Doppelbindungen. Beispielhaft und vorzugsweise seien genannt: Ethen-1,1-diyl, Ethen-1,2-diyl, Propen-1,1-diyl, Propen-1,2-diyl, Propen-1,3-diyl, But-1-en-1,4-diyl, But-1-en-1,3-diyl, But-2-en-1,4-diyl, Buta-1,3-dien-1,4-diyl, Pent-2-en-1,5-diyl, Hex-3-en-1,6-diyl und Hexa-2,4-dien-1,6-diyl.
(C₂-C₇)-Alkindiyl steht im Rahmen der Erfindung für einen geradkettigen oder verzweigten divalenten Alkinylrest mit 2 bis 7 Kohlenstoffatomen und bis 3 Dreifachbindungen. Bevorzugt ist ein geradkettiger Alkindiylrest mit 2 bis 6 Kohlenstoffatomen und bis zu 2 Dreifachbindungen. Beispielhaft und vorzugsweise seien genannt: Ethin-1,2-diyl, Propin-1,3-diyl, But-1-in-1,4-diyl, But-1-in-1,3-diyl, But-2-in-1,4-diyl, Pent-2-in-1,5-diyl, Pent-2-in-1,4-diyl und Hex-3-in-1,6-diyl.
(C₁-C₆)-Alkoxy und (C₁-C₄)-Alkoxy stehen im Rahmen der Erfindung für einen geradkettigen oder verzweigten Alkoxyrest mit 1 bis 6 bzw. 1 bis 4 Kohlenstoffatomen. Bevorzugt ist ein geradkettiger oder verzweigter Alkoxyrest mit 1 bis 4 Kohlenstoffatomen. Beispielhaft und vorzugsweise seien genannt: Methoxy, Ethoxy, n-Propoxy, Isopropoxy, n-Butoxy, *tert*.-Butoxy, n-Pentoxy und n-Hexoxy.
(C₁-C₄)-Alkoxycarbonyl steht im Rahmen der Erfindung für einen geradkettigen oder verzweigten Alkoxyrest mit 1 bis 4 Kohlenstoffatomen, der über eine Carbonylgruppe verknüpft ist. Beispielhaft und vorzugsweise seien genannt: Methoxycarbonyl, Ethoxycarbonyl, n-Propoxycarbonyl, Isopropoxycarbonyl und *tert*.-Butoxycarbonyl.

Halogen schließt im Rahmen der Erfindung Fluor, Chlor, Brom und Iod ein. Bevorzugt sind Chlor, Fluor oder Brom, besonders bevorzugt Fluor oder Chlor.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem oder zwei Substituenten.

Wenn ein Rest in den erfindungsgemäßen Verbindungen mehrfach mit Fluor substituiert sein kann, so schließt dies im Rahmen der vorliegenden Erfindung eine Perfluor-Substitution mit ein.

Ebenfalls offenbart sind nicht erfindungsgemäße Verbindungen der Formel (I), in welcher
- A: für eine Gruppe der Formel 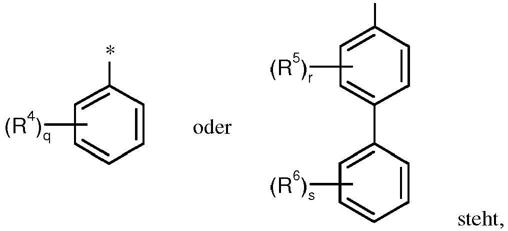
worin * die Verknüpfungsstelle mit der Gruppe D bedeutet,
- D: für eine Bindung oder (C₁-C₇)-Alkandiyl steht,

R¹, R⁴, R⁵ und R⁶ unabhängig voneinander für einen Substituenten ausgewählt aus der Reihe Fluor, Chlor, Brom, (C₁-C₄)-Alkyl, Trifluormethyl, (C₁-C₄)-Alkoxy und Trifluormethoxy stehen,
n, q, r und s unabhängig voneinander jeweils für die Zahl 0, 1 oder 2 stehen,
   wobei für den Fall, dass R¹, R⁴, R⁵ oder R⁶ mehrfach auftreten, ihre Bedeutungen jeweils gleich oder verschieden sein können,
R² und R³ jeweils für Fluor stehen,
und
o und p unabhängig voneinander jeweils für die Zahl 0 oder 1 stehen,
sowie ihre Salze, Solvate und Solvate der Salze.

Gegenstand der vorliegenden Erfindung sind Verbindungen der Formel (I-A) 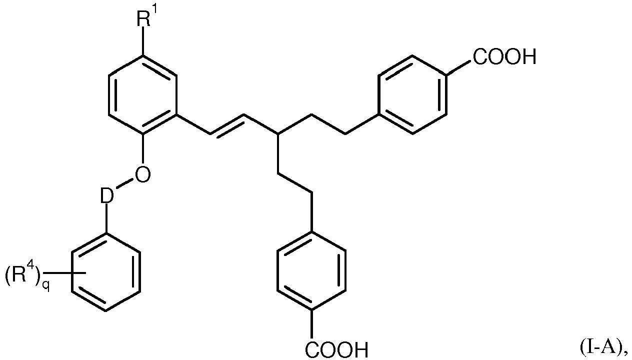 in welcher
- D: für (C₁-C₇)-Alkandiyl steht,
- R¹: für Wasserstoff oder Fluor steht,
- R⁴: für einen Substituenten ausgewählt aus der Reihe Fluor, Chlor, Methyl, *tert*.-Butyl, Trifluormethyl, Methoxy und Trifluormethoxy steht,
und
- q: für die Zahl 0, 1 oder 2 steht, wobei für den Fall, dass der Substituent R⁴ zweifach auftritt, seine Bedeutungen gleich oder verschieden sein können,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Offenbart ist ein Verfahren zur Herstellung der erfindungsgemäßen Verbindungen der Formel (I), dadurch gekennzeichnet, dass man Verbindungen der Formel (II) 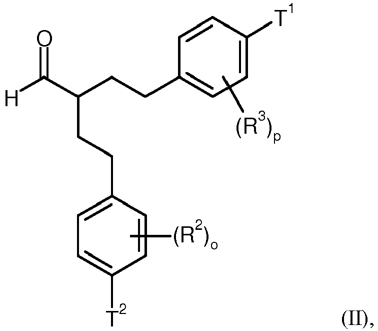
in welcher R², R³, o und p jeweils die oben angegebenen Bedeutungen haben und
T¹ und T² gleich oder verschieden sind und für (C₁-C₄)-Alkoxycarbonyl stehen, entweder
   [A] in einem inerten Lösungsmittel in Gegenwart einer Base mit einer Verbindung der Formel (III-A) 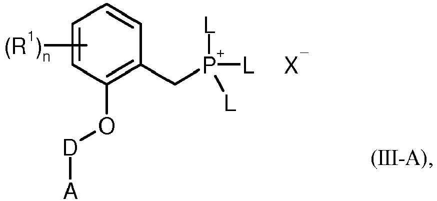 in welcher A, D, R¹ und n jeweils die oben angegebenen Bedeutungen haben und
      - L: für Phenyl oder o-, m- oder p-Tolyl
      und
      - X: für Halogenid oder Tosylat steht,
      zu Verbindungen der Formel (IV-A) 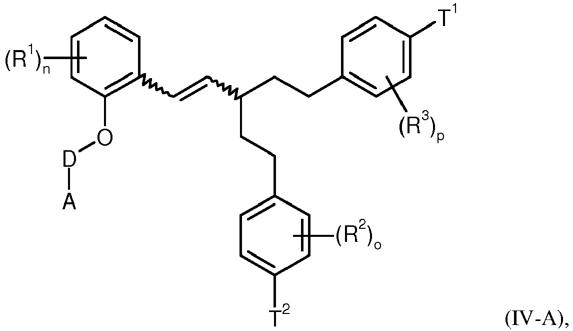 in welcher A, D, R¹, R², R³, n, o, p, T¹ und T² jeweils die oben angegebenen Bedeutungen haben,
      umsetzt
      oder
   [B] in einem inerten Lösungsmittel in Gegenwart einer Base mit einer Verbindung der Formel (III-B) 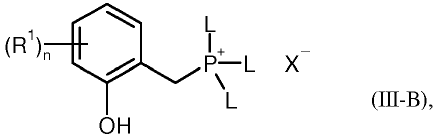 in welcher R¹, n, L und X jeweils die oben angegebenen Bedeutungen haben, zunächst zu Verbindungen der Formel (IV-B) 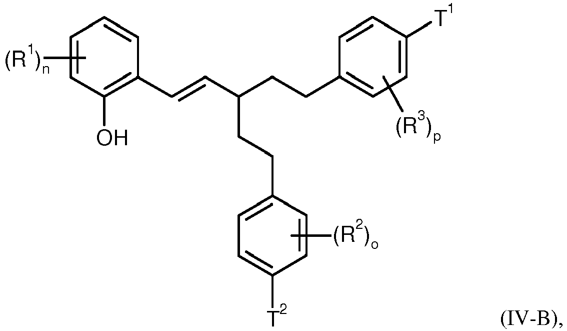 in welcher R¹, R², R³, n, o, p, T¹ und T² jeweils die oben angegebenen Bedeutungen haben, umsetzt und diese anschließend in einem inerten Lösungsmittel in Gegenwart einer Base mit einer Verbindung der Formel (V)

      A-D-Q (V),

      in welcher A und D die oben angegebenen Bedeutungen haben und
      - Q: für eine Abgangsgruppe, wie beispielsweise Halogen, Tosylat oder Mesylat, steht,
      zu Verbindungen der Formel (IV-C) 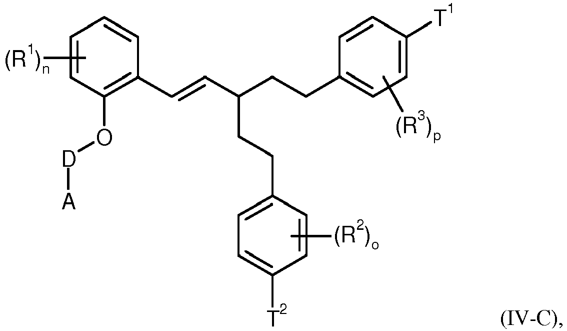 in welcher A, D, R¹, R², R³, n, o, p, T¹ und T² jeweils die oben angegebenen Bedeutungen haben,
      kuppelt
und die resultierenden Verbindungen der Formel (IV-A) bzw. (IV-C) dann durch Hydrolyse der Ester-Gruppen T¹ und T² in die Dicarbonsäuren der Formel (I) überführt
und die Verbindungen der Formel (I) gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

Inerte Lösungsmittel für die Verfahrensschritte (II) + (III-A) → (IV-A) und (II) + (III-B) → (IV-B) sind beispielsweise Ether wie Diethylether, Tetrahydrofuran, Glykoldimethylether oder Diethylenglykoldimethylether, oder Kohlenwasserstoffe wie Benzol, Toluol, Xylol, Pentan, Hexan, Heptan, Cyclohexan oder Erdölfraktionen, oder Gemische dieser Lösungsmittel. Bevorzugt wird Tetrahydrofuran im Gemisch mit Hexan verwendet.

Als Basen für diese Verfahrensschritte sind die für eine Wittig-Reaktion üblichen Basen geeignet. Hierzu zählen insbesondere starke Basen wie *n-, sek.-* oder *tert*.-Butyllithium, Lithiumdiisopropylamid (LDA) oder Lithium-, Natrium- oder Kalium-bis(trimethylsilyl)amid. Bevorzugt ist *n*-Butyllithium.

Die Umsetzungen (II) + (III-A) → (IV-A) und (II) + (III-B) → (IV-B) werden im Allgemeinen in einem Temperaturbereich von -78°C bis +20°C, bevorzugt bei -20°C bis +10°C durchgeführt.

Gegebenenfalls bei der Reaktion (II) + (III-A) → (IV-A) anfallende *cisltrans*-Gemische der Verbindung (IV-A) können auf dieser oder auf der nachfolgenden Stufe der Verbindung (I) nach geläufigen Methoden, beispielsweise durch Chromatographie, getrennt werden. Die Reaktion (II) + (III-B) → (IV-B) verläuft in der Regel *trans*-selektiv.

Inerte Lösungsmittel für den Verfahrensschritt (IV-B) + (V) → (IV-C) sind beispielsweise Ether wie Diethylether, Dioxan, Tetrahydrofuran, Glykoldimethylether oder Diethylenglykoldimethylether, Kohlenwasserstoffe wie Benzol, Toluol, Xylol, Pentan, Hexan, Heptan, Cyclohexan oder Erdölfraktionen, oder andere Lösungsmittel wie Acetonitril, Dimethylformamid, Dimethylsulfoxid, *N,N*'-Dimethylpropylenharnstoff (DMPU) oder *N*-Methylpyrrolidon (NMP). Ebenso ist es möglich, Gemische der genannten Lösungsmittel einzusetzen. Bevorzugt werden Acetonitril, Dimethylformamid, Dioxan oder Toluol verwendet.

Für diesen Verfahrensschritt geeignete Basen sind insbesondere Natrium-, Kalium- oder Caesiumcarbonat, Natrium- oder Kaliumhydrid, Lithiumdiisopropylamid oder *n*-Butyllithium. Bevorzugt wird Kalium- oder Caesiumcarbonat oder Natriumhydrid verwendet.

Die Reaktion (IV-B) + (V) → (IV-C) wird im Allgemeinen in einem Temperaturbereich von +20°C bis +150°C, bevorzugt bei +50°C bis +120°C durchgeführt.

Die Hydrolyse der Ester-Gruppen T¹ und T² in den Verfahrensschritten (IV-A) → (I) und (IV-C) → (I) erfolgt nach üblichen Methoden, indem man die Ester in inerten Lösungsmitteln mit Säuren oder Basen behandelt, wobei bei letzterem die zunächst entstehenden Salze durch Behandeln mit Säure in die freien Carbonsäuren überführt werden. Im Falle der *tert*.-Butylester erfolgt die Esterspaltung bevorzugt mit Säuren.

Bei unterschiedlichen Gruppen T¹ und T² kann die Hydrolyse gegebenenfalls simultan in einer Eintopf-Reaktion oder in zwei separaten Reaktionsschritten durchgeführt werden.

Als inerte Lösungsmittel eignen sich für diese Reaktionen Wasser oder die für eine Esterspaltung üblichen organischen Lösungsmittel. Hierzu gehören bevorzugt Alkohole wie Methanol, Ethanol, n-Propanol, Isopropanol, n-Butanol oder *tert*.-Butanol, oder Ether wie Diethylether, Tetrahydrofuran, Dioxan oder Glykoldimethylether, oder andere Lösungsmittel wie Aceton, Dichlormethan, Dimethylformamid oder Dimethylsulfoxid. Ebenso ist es möglich, Gemische der genannten Lösungsmittel einzusetzen. Im Falle einer basischen Ester-Hydrolyse werden bevorzugt Gemische von Wasser mit Dioxan, Tetrahydrofuran, Methanol und/oder Ethanol eingesetzt. Im Falle der Umsetzung mit Trifluoressigsäure wird bevorzugt Dichlormethan und im Falle der Umsetzung mit Chlorwasserstoff bevorzugt Tetrahydrofuran, Diethylether, Dioxan oder Wasser verwendet.

Als Basen sind die üblichen anorganischen Basen geeignet. Hierzu gehören bevorzugt Alkali- oder Erdalkalihydroxide wie beispielsweise Natrium-, Lithium-, Kalium- oder Bariumhydroxid, oder Alkali- oder Erdalkalicarbonate wie Natrium-, Kalium- oder Calciumcarbonat. Besonders bevorzugt sind Natrium-, Kalium- oder Lithiumhydroxid.

Als Säuren eignen sich für die Esterspaltung im Allgemeinen Schwefelsäure, Chlorwasserstoff/ Salzsäure, Bromwasserstoff/Bromwasserstoffsäure, Phosphorsäure, Essigsäure, Trifluoressigsäure, Toluolsulfonsäure, Methansulfonsäure oder Trifluormethansulfonsäure oder deren Gemische gegebenenfalls unter Zusatz von Wasser. Bevorzugt sind Chlorwasserstoff oder Trifluoressigsäure im Falle der *tert*.-Butylester und Salzsäure im Falle der Methylester.

Die Esterspaltung erfolgt im Allgemeinen in einem Temperaturbereich von 0°C bis +100°C, bevorzugt bei +20°C bis +60°C.

Die genannten Umsetzungen können bei normalem, erhöhtem oder bei erniedrigtem Druck durchgeführt werden (z.B. von 0.5 bis 5 bar). Im Allgemeinen arbeitet man jeweils bei Normaldruck.

Die Aldehyde der Formel (II) können in Analogie zu literaturbekannten Verfahren beispielsweise über eine zweifache Alkylierung von Malonsäurediallylester mit Verbindungen der Formeln (VI) und (VII) 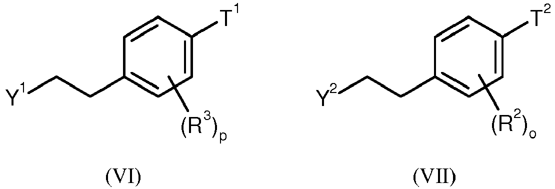
in welchen R², R³, o, p, T¹ und T² jeweils die oben angegebenen Bedeutungen haben und
Y¹ und Y² gleich oder verschieden sind und für eine Abgangsgruppe, wie beispielsweise Halogen, Mesylat oder Tosylat, stehen,
zu Verbindungen der Formel (VIII) 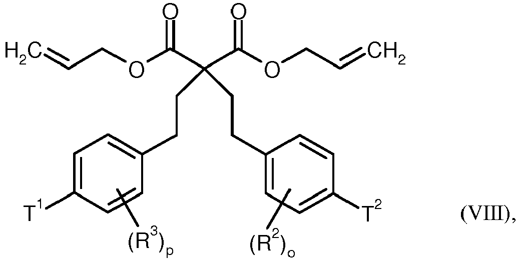 in welcher R², R³, o, p, T¹ und T² jeweils die oben angegebenen Bedeutungen haben,
anschließende Esterspaltung zu Verbindungen der Formel (IX) 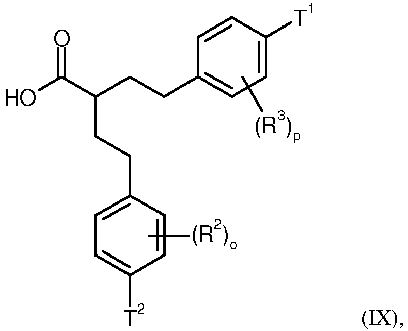
in welcher R², R³, o, p, T¹ und T² jeweils die oben angegebenen Bedeutungen haben,
und nachfolgende Reduktion der Carbonsäure-Gruppierung hergestellt werden (siehe auch nachfolgende Reaktionsschemata 1 und 2).

Die Verbindungen der Formeln (III-A) und (III-B) können nach literaturüblichen Verfahren durch Umsetzung von Verbindungen der Formel (X-A) bzw. (X-B) 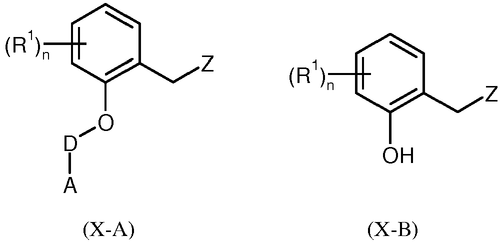 in welchen A, D, R¹ und n jeweils die oben angegebenen Bedeutungen haben und
- Z: für eine Abgangsgruppe, wie beispielsweise Halogen oder Tosylat, oder für Hydroxy steht, mit beispielsweise Triphenylphosphin bzw. (im Falle von Z = OH) Triphenylphosphin-Hydrobromid erhalten werden (siehe auch nachfolgendes Reaktionsschema 3).

Die Verbindungen der Formeln (V), (VI), (VII), (X-A) und (X-B) sind kommerziell erhältlich, literaturbekannt oder können in Analogie zu literaturbekannten Verfahren hergestellt werden. Die Verbindungen der Formel (X-A) können beispielsweise analog zum Verfahrensschritt (IV-B) + (V) → (IV-C) durch Reaktion von Verbindungen der Formel (X-B), worin Z für Hydroxy steht, mit einer Verbindung der Formel (V) erhalten werden (siehe Reaktionsschema 3).

Eine Trennung der erfindungsgemäßen Verbindungen in die entsprechenden Enantiomere und/oder Diastereomere kann gegebenenfalls, je nach Zweckmäßigkeit, auch bereits auf der Stufe der Verbindungen (IV-A), (IV-B), (IV-C) oder (IX) erfolgen, welche dann in separierter Form entsprechend der zuvor beschriebenen Verfahrenssequenz weiter umgesetzt werden. Eine solche Auftrennung der Stereoisomeren läßt sich nach üblichen, dem Fachmann bekannten Methoden durchführen; vorzugsweise werden chromatographische Verfahren oder eine Trennung über diastereomere Salze verwendet.

Die Herstellung der erfindungsgemäßen Verbindungen kann durch die folgenden Syntheseschemata veranschaulicht werden:

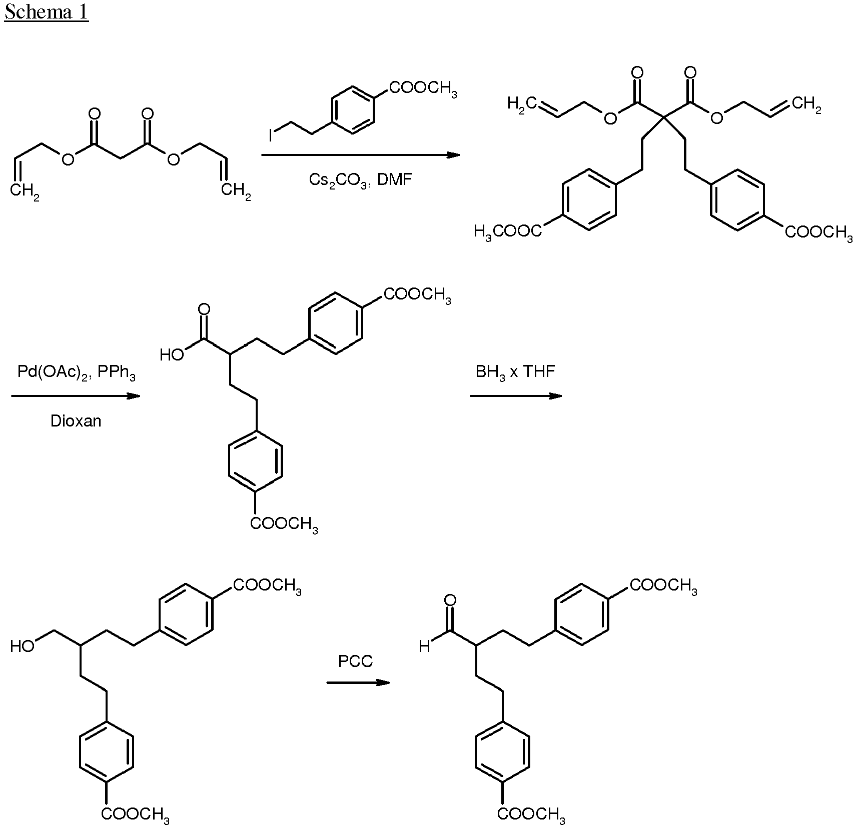

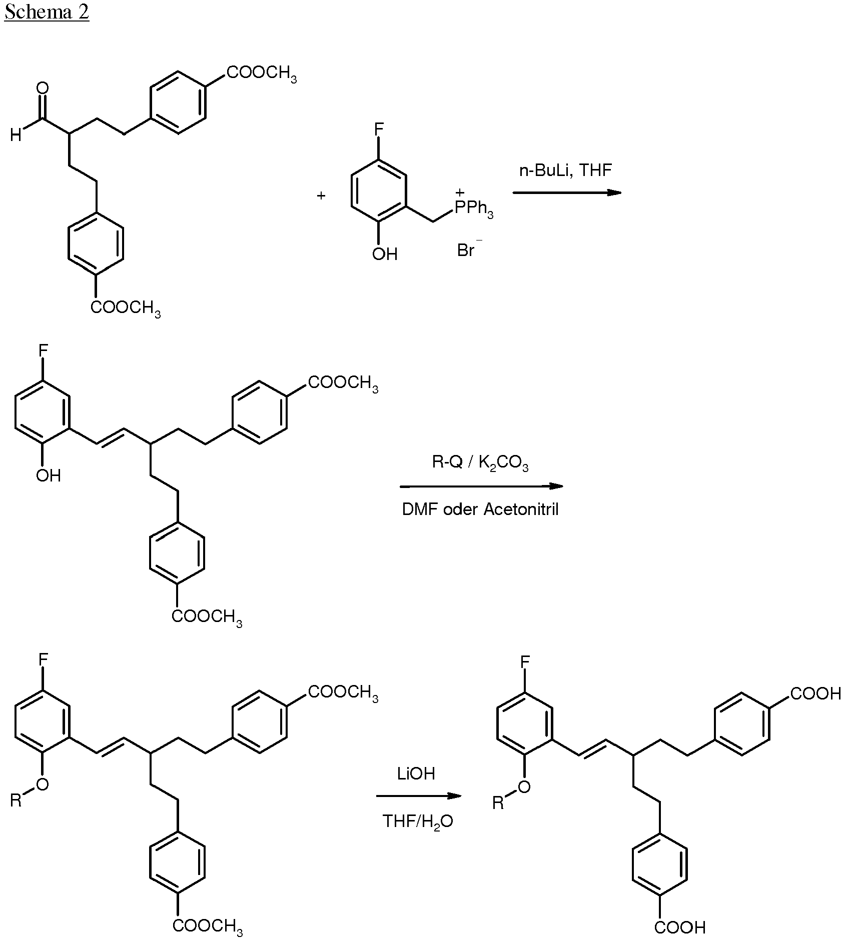

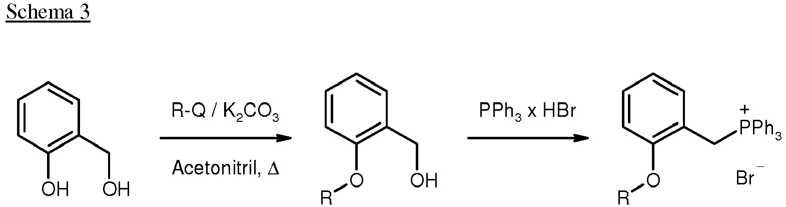

[Abkürzungen: Ac = Acetyl; BH₃ x THF = Boran-Tetrahydrofuran-Komplex; Bu = Butyl; DMF = Dimethylformamid; PCC = Pyridiniumchlorochromat; Ph = Phenyl; Q = Abgangsgruppe, z.B. Halogen; THF = Tetrahydrofuran].

Die erfindungsgemäßen Verbindungen besitzen wertvolle pharmakologische Eigenschaften und können zur Vorbeugung und Behandlung von Erkrankungen bei Menschen und Tieren verwendet werden.

Als besonderes und überraschendes Merkmal weisen die Verbindungen der vorliegenden Erfindung vorteilhafte pharmakokinetische Eigenschaften wie beispielsweise eine erhöhte Bioverfügbarkeit und/oder eine verlängerte Wirkdauer nach oraler Gabe auf.

Die erfindungsgemäßen Verbindungen führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte Aktivierung der löslichen Guanylatcyclase und einen intrazellulären cGMP-Anstieg vermittelt.

Die erfindungsgemäßen Verbindungen können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, pulmonaler Hypertonie, peripheren und kardialen Gefäßerkrankungen, Arrhythmien, zur Behandlung von thromboembolischen Erkrankungen und Ischämien wie Myokardinfarkt, Hirnschlag, transistorischen und ischämischen Attacken, peripheren Durchblutungsstörungen, zur Verhinderung von Restenosen wie nach Thrombolysetherapien, percutan-transluminalen Angioplastien (PTA), percutan-transluminalen Koronarangioplastien (PTCA) und Bypass sowie zur Behandlung von Arteriosklerose, asthmatischen Erkrankungen, Krankheiten des Urogenitalsystems wie beispielsweise Prostatahypertrophie, erektile Dysfunktion, weibliche sexuelle Dysfunktion und Inkontinenz, von Osteoporose, Glaukom und Gastroparese eingesetzt werden.

Außerdem können die erfindungsgemäßen Verbindungen zur Behandlung von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, CREST-Syndrom, Erythematose, Onychomykose sowie von rheumatischen Erkrankungen verwendet werden.

Ferner eignen sich die erfindungsgemäßen Verbindungen zur Behandlung von Respiratory Distress-Syndromen und chronisch-obstruktiven Atemwegserkrankungen (COPD), von akuter und chronischer Niereninsuffizienz sowie zur Förderung der Wundheilung.

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei SituationeuKrankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- NO-unabhängige, jedoch Häm-abhängige Stimulatoren der Guanylatcyclase, wie insbesondere die in WO 00/06568, WO 00/06569, WO 02/42301 und WO 03/095451 beschriebenen Verbindungen;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise BAY 59-7939, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT- Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale und die intravenöse Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- abs.: Absolut
- aq.: Wässrig
- Bsp.: Beispiel
- CI: chemische Ionisation (bei MS)
- DC: Dünnschichtchromatographie
- DCI: direkte chemische Ionisation (bei MS)
- DMF: Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- eq.: Äquivalent(e)
- ESI: Elektrospray-Ionisation (bei MS)
- GC: Gaschromatographie
- h: Stunde(n)
- HPLC: Hochdruck-, Hochleistungsflüssigchromatographie
- LC-MS: Flüssigchromatographie-gekoppelte Massenspektroskopie
- min: Minute(n)
- MS: Massenspektroskopie
- NMR: Kernresonanzspektroskopie
- R_{f}: Retentionsindex (bei DC)
- RT: Raumtemperatur
- Rₜ: Retentionszeit (bei HPLC)
- THF: Tetrahydrofuran
- UV: Ultraviolett-Spektroskopie
- v/v: Volumen zu Volumen-Verhältnis (einer Lösung)

### LC/MS-Methoden:

### Methode 1 (LC-MS)

Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 2 (LC-MS)

Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 3 (LC-MS)

Instrument: Micromass Platform LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 4 (LC-MS)

Instrument: Micromass Quattro LCZ mit HPLC Agilent Serie 1100; Säule: Phenomenex Synergi 2µ Hydro-RP Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 208-400 nm.

### Methode 5 (LC-MS)

Instrument: Micromass Platform LCZ mit HPLC Agilent Serie 1100; Säule: Thermo Hypersil GOLD 3µ 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

### Methode 6 (LC-MS)

Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%-ige Ameisensäure / 1, Eluent B: Acetonitril + 500 µl 50%-ige Ameisensäure / 1; Gradient: 0.0 min 10% B → 7.0 min 95% B → 9.0 min 95% B; Fluss: 0.0 min 1.0 ml/min → 7.0 min 2.0 ml/min → 9.0 min 2.0 ml/min; Ofen: 35°C; UV-Detektion: 210 nm.

### Methode 7 (LC-MS)

Gerätetyp MS: Micromass ZQ; Gerätetyp HPLC: HP 1100 Series; UV DAD; Säule: Phenomenex Gemini 3µ 30 mm x 3.00 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 8 (LC-MS)

Instrument: Micromass Quattro LCZ mit HPLC Agilent Serie 1100; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 208-400 nm.

### Methode 9 (LC-MS)

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### GC/MS-Methoden:

### Methode 1 (GC-MS)

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (1.7 min halten).

### Methode 2 (GC-MS)

Instrument: Micromass GCT, GC6890; Säule: Restek RTX-35MS, 30 m x 250 µm x 0.25 µm; konstanter Fluss mit Helium: 0.88 ml/min; Ofen: 60°C; Inlet: 250°C; Gradient: 60°C (0.30 min halten), 50°C/min → 120°C, 16°C/min → 250°C, 30°C/min → 300°C (8.7 min halten).

### HPLC-Methoden:

### Methode 1 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 9 min 90% B → 9.2 min 2% B → 10 min 2% B; Fluss: 0.75 ml/min; Säulentemperatur: 30°C; UV-Detektion: 210 nm.

### Methode 2 (HPLC)

Instrument: HP 1100 mit DAD-Detektion; Säule: Kromasil 100 RP-18, 60 mm x 2.1 mm, 3.5 µm; Eluent A: 5 ml HClO₄ (70%-ig) / 1 Wasser, Eluent B: Acetonitril; Gradient: 0 min 2% B → 0.5 min 2% B → 4.5 min 90% B → 15 min 90% B → 15.2 min 2% B → 16 min 2% B; Fluss: 0.75 ml/min; Säulentemperatur: 30°C; UV-Detektion: 210 nm.

### Ausgangsverbindungen und Intermediate:

### Beispiel 1A

### (5 -Brompentyl)benzol

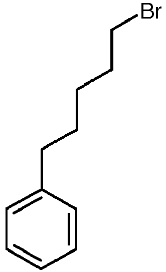

Eine Lösung von 416.7 ml (1.83 mol) 48%-iger Bromwasserstoffsäure wird bei 0°C mit 50 g (0.304 mol) 5-Phenylpentan-1-ol versetzt und 30 min bei 0°C nachgerührt. Anschließend wird die Reaktionslösung 12 Stunden bei 100°C gerührt. Nach vollständiger Umsetzung wird der Ansatz auf Raumtemperatur abgekühlt und mit 200 ml Essigsäureethylester versetzt. Nach Extraktion wird die organische Phase abgetrennt, mit gesättigter Natriumhydrogencarbonat-Lösung gewaschen und über Natriumsulfat getrocknet. Nach Filtration wird das Filtrat bis zur Trockene eingeengt. Das erhaltene Rohprodukt wird durch Flash-Chromatographie an Kieselgel (Laufmittel: Cyclohexan) gereinigt. Es werden 59.4 g (0.26 mol, 86% d. Th.) einer farblosen Flüssigkeit erhalten.

¹H-NMR (300 MHz, CDCl₃, δ/ppm): 7.32-7.22 (2H, m), 7.21-7.11 (3H, m), 3.40 (2H, t), 2.61 (2H, t), 1.97-1.81 (2H, m), 1.72-1.58 (2H, m), 1.56-1.39 (2H, m).

MS (CI): m/z = 226 (M⁺).

### Beispiel 2A

### 4-Fluor-2-(hydroxymethyl)phenol

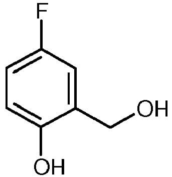

Unter Sauerstoffausschluss werden 27.1 g (159.28 mmol) 5-Fluor-2-hydroxybenzoesäuremethylester in 500 ml trockenem THF vorgelegt und auf 0°C abgekühlt. Anschließend tropft man langsam unter Kühlung 238 ml (238 mmol) einer 1 M Lösung von Lithiumaluminiumhydrid in THF hinzu und rührt 1 Stunde bei 0°C und dann über Nacht bei RT nach. Nach vollständiger Umsetzung wird der Ansatz mit gesättigter Ammoniumchlorid-Lösung versetzt und in Dichlormethan aufgenommen. Die organische Phase wird abgetrennt und über Natriumsulfat getrocknet. Nach Filtration wird das Lösungsmittel im Vakuum entfernt. Das Rohprodukt wird über Kieselgel chromatographisch (Laufmittel: Cyclohexan/Essigsäureethylester 20:1) gereinigt. Es werden 18.0 g (126.6 mmol, 79% d. Th.) eines farblosen Feststoffes isoliert.

¹H-NMR (400 MHz, DMSO-d₆, δ/ppm): 9.32 (1H, s), 7.06-7.03 (1H, m), 6.86-6.81 (1H, m), 6.74-6.71 (1H, m), 5.09 (1H, t), 4.45 (2H, d).

### Beispiel 3A

### (5-Fluor-2-hydroxybenzyl)(triphenyl)phosphoniumbromid

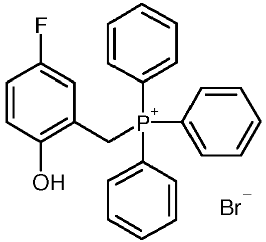

18.6 g (130.87 mmol) 4-Fluor-2-(hydroxymethyl)phenol und 42.67 g (124.32 mmol) Triphenylphosphoniumhydrobromid werden in 186 ml Acetonitril 3 h lang unter Rückfluss gerührt. Nach dem Abkühlen wird der entstandene Niederschlag abgesaugt und getrocknet. Es werden 58 g (124 mmol, 100% d. Th.) der Titelverbindung erhalten.

¹H-NMR (400 MHz, DMSO-d₆, δ/ppm): 9.82 (1H, s), 7.95-7.84 (3H, m), 7.79-7.62 (12H, m), 7.02-6.91 (1H, m), 6.75-6.67 (1H, m), 6.66-6.58 (1H, m), 4.90 (2H, d).

### Beispiel 4A

### 4-(2-Iodethyl)benzoesäuremethylester

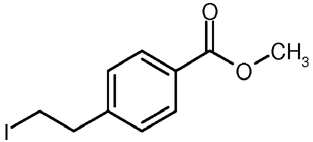

70 g (352.4 mmol) 4-(2-Chlorethyl)benzoesäuremethylester [CAS Reg.-Nr. 65787-72-6] und 146.2 g (880.9 mmol) Kaliumiodid werden in 800 ml Acetonitril suspendiert und drei Tage unter Rückfluss gerührt. Nach vollständigem Umsatz wird die Reaktionslösung abgekühlt, filtriert und das Filtrat im Vakuum bis zur Trockene eingeengt. Der resultierende Rückstand wird mittels Flash-Chromatographie an Kieselgel gereinigt (Laufmittel: Petrolether/Essigsäureethylester 30:1 → 20:1). Es werden 101 g (348.3 mmol, 98.8% d. Th.) eines gelblichen Öls erhalten.

LC-MS (Methode 7): Rₜ = 2.66 min; m/z = 291 (M+H)⁺.

### Beispiel 5A

Bis{2-[4-(methoxycarbonyl)phenyl]ethyl}-malonsäurediallylester

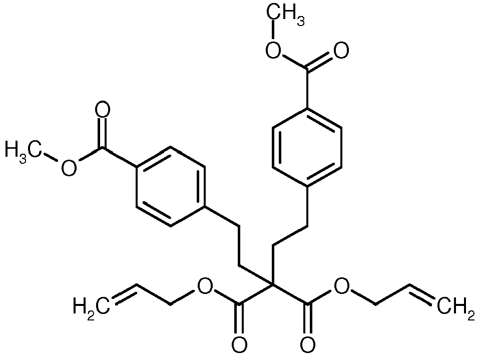

Eine Lösung von 10 g (54.3 mmol) Malonsäurediallylester und 47.25 g (80% Reinheit, 130.3 mmol) 4-(2-Iodethyl)benzoesäuremethylester in 100 ml DMF wird bei Raumtemperatur mit 61.9 g (190.02 mmol) Caesiumcarbonat versetzt und anschließend über Nacht bei Raumtemperatur gerührt. Nach vollständigem Umsatz wird die Reaktionslösung bis zur Trockene eingeengt und der Rückstand in 100 ml Wasser und 100 ml Diethylether aufgenommen. Die wässrige Phase wird fünfmal mit Diethylether extrahiert und die vereinigten organischen Phasen mit gesättigter Natriumchlorid-Lösung gewaschen und über Magnesiumsulfat getrocknet. Nach Filtration wird das Lösungsmittel im Vakuum entfernt und der Rückstand durch Flash-Chromatographie an Kieselgel gereinigt (Laufmittel: Petrolether/Essigsäureethylester 10:1 → 5:1). Es werden 22.64 g (44.52 mmol, 82% d. Th.) eines gelblichen Öls erhalten.

LC-MS (Methode 7): Rₜ = 3.14 min; m/z = 509 (M+H)⁺.

### Beispiel 6A

### 4-[4-(Methoxycarbonyl)phenyl]-2-{2-[4-(methoxycarbonyl)phenyl]ethyl}butansäure

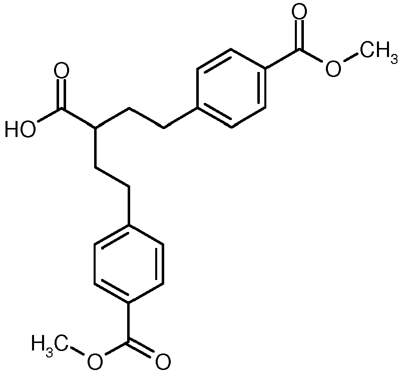

Eine Lösung von 22.64 g (44.52 mmol) Bis{2-[4-(methoxycarbonyl)phenyl]ethyl}-malonsäurediallylester, 0.82 g (3.12 mmol) Triphenylphosphin und 200 mg Palladiumacetat in 250 ml Dioxan wird bei Raumtemperatur mit einer Lösung von 20.48 ml (146.9 mmol) Triethylamin und 4.2 ml (111.29 mmol) Ameisensäure in 50 ml Dioxan versetzt. Das Reaktionsgemisch wird anschließend über Nacht bei 100°C gerührt. Nach vollständigem Umsatz wird die Reaktionslösung abgekühlt und das Lösungsmittel im Vakuum entfernt. Der erhaltene Rückstand wird in Wasser und Essigsäureethylester aufgenommen. Die wässrige Phase wird dreimal mit Essigsäureethylester extrahiert, die vereinigten organischen Phasen mit gesättigter Natriumchlorid-Lösung gewaschen und über Magnesiumsulfat getrocknet. Nach Filtration wird das Lösungsmittel im Vakuum entfernt und der Rückstand durch Flash-Chromatographie an Kieselgel gereinigt (Laufmittel: Cyclohexan/ Essigsäureethylester 4:1). Es werden 10.4 g (27.1 mmol, 61% d. Th.) eines farblosen Feststoffs erhalten.

¹H-NMR (400 MHz, DMSO-d₆, δ/ppm): 12.30 (1H, s), 7.86 (4H, d), 7.31 (4H, d), 3.84 (6H, s), 2.72-2.56 (4H, m), 2.29-2.18 (1H, m), 1.92-1.69 (4H, m).

LC-MS (Methode 7): Rₜ = 2.55 min; m/z = 385 (M+H)⁺.

### Beispiel 7A

### Dimethyl 4,4'-[3-(hydroxymethyl)pentan-1,5-diyl] dibenzoat

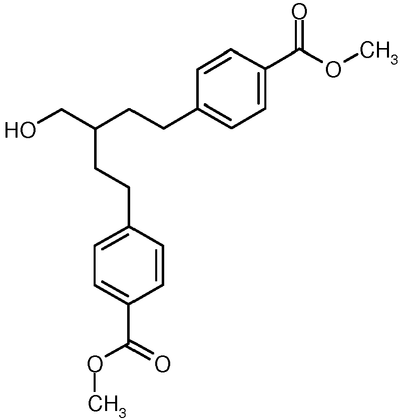

Zu einer Lösung von 10.4 g (27.1 mmol) 4-[4-(Methoxycarbonyl)phenyl]-2-{2-[4-(methoxycarbonyl)phenyl]ethyl}butansäure in 260 ml THF werden 54.1 ml (54.1 mmol) einer 1 M Boran-THF-Komplex-Lösung bei -10°C zugetropft. Anschließend wird das Reaktionsgemisch auf 0°C erwärmt und 4 h bei dieser Temperatur nachgerührt. Nach vollständiger Umsetzung wird das Reaktionsgemisch mit gesättigter Ammoniumchlorid-Lösung versetzt, mit Wasser und Essigsäureethylester verdünnt und die wässrige Phase zweimal mit Essigsäureethylester extrahiert. Die vereinigten organischen Phasen werden über Magnesiumsulfat getrocknet und im Vakuum vom Lösungsmittel befreit. Es werden 8.73 g (23.56 mmol, 87% d. Th.) eines farblosen Öls erhalten.

LC-MS (Methode 7): Rₜ = 2.62 min; m/z = 371 (M+H)⁺.

### Beispiel 8A

### Dimethyl 4,4'-(3-formylpentan-1,5-diyl)dibenzoat

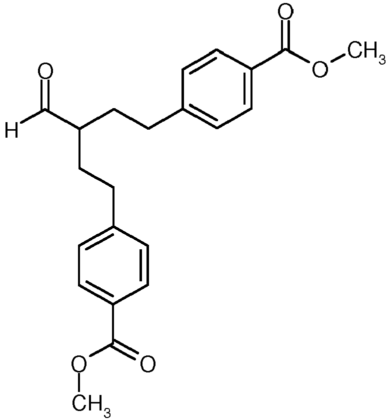

Eine Lösung von 8.73 g (23.6 mmol) Dimethyl 4,4'-[3-(hydroxymethyl)pentan-1,5-diyl]dibenzoat in 280 ml Dichlormethan wird mit 6.1 g (28.3 mmol) Pyridiniumchlorochromat (PCC) versetzt und 12 h bei Raumtemperatur gerührt. Nach vollständigem Umsatz werden ca. 10 g Kieselgel zugegeben und das Lösungsmittel im Vakuum bis zur Trockene entfernt. Der Rückstand wird durch Flash-Chromatographie an Kieselgel gereinigt (Laufmittel: Cyclohexan/Essigsäureethylester 4:1). Es werden 7.08 g (19.22 mmol, 81.5% d. Th.) eines farblosen Öls erhalten.

LC-MS (Methode 2): Rₜ = 2.61 min; m/z = 369 (M+H)⁺.

### Beispiel 9A

### 4-[(4E)-5-(5-Fluor-2-hydroxyphenyl)-3-{2-[4-(methoxycarbonyl)phenyl]ethyl}pent-4-en-1-yl]-benzoesäuremethylester

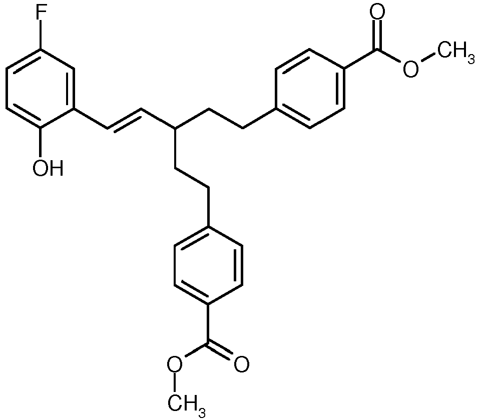

Zu einer Lösung von 1479 mg (3.2 mmol) (5-Fluor-2-hydroxybenzyl)(triphenyl)phosphonium-bromid in 40 ml THF werden bei 0°C 2.95 ml (7.39 mmol) einer 2.5 M Lösung von n-Butyllithium in Hexan langsam zugetropft. Das Reaktionsgemisch wird 45 min bei dieser Temperatur nachgerührt. Anschließend werden bei 0°C 1080 mg (2.64 mmol) Dimethyl 4,4'-(3-formylpentan-1,5-diyl)dibenzoat in 10 ml THF langsam zudosiert. Die Reaktionslösung wird 5 h bei 0°C nachgerührt, dann mit gesättigter Ammoniumchlorid-Lösung versetzt und mit Wasser und Essigsäureethylester verdünnt. Die organische Phase wird abgetrennt und die wässrige Phase noch zweimal mit Essigsäureethylester extrahiert. Die vereinigten organischen Phasen werden über Natriumsulfat getrocknet, filtriert und das Lösungsmittel bis zur Trockene entfernt. Das erhaltene Rohprodukt wird durch Flash-Chromatographie an Kieselgel gereinigt (Laufmittel: Cyclohexan/Essigsäureethylester 4:1). Es werden 529 mg (1.11 mmol, 42% d. Th.) eines farblosen Öls erhalten.

LC-MS (Methode 2): Rₜ = 3.01 min; m/z = 477 (M+H)⁺.

### Beispiel 10A

### 4-[(4E)-5-(2-Hydroxyphenyl)-3-{2-[4-(methoxycarbonyl)phenyl]ethyl}pent-4-en-1-yl]benzoesäuremethylester

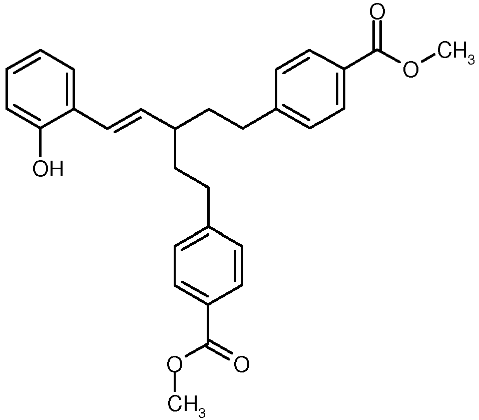

Zu einer Lösung von 1422 mg (3.2 mmol) (2-Hydroxybenzyl)(triphenyl)phosphoniumbromid in 40 ml THF werden bei 0°C 2.95 ml (7.39 mmol) einer 2.5 M Lösung von n-Butyllithium in Hexan langsam zugetropft. Das Reaktionsgemisch wird 45 min bei dieser Temperatur nachgerührt. Anschließend werden bei 0°C 1080 mg (2.64 mmol) Dimethyl 4,4'-(3-formylpentan-1,5-diyl)dibenzoat in 10 ml THF langsam zudosiert. Die Reaktionslösung wird 5 h bei 0°C nachgerührt, dann mit gesättigter Ammoniumchlorid-Lösung versetzt und mit Wasser und Essigsäureethylester verdünnt. Die organische Phase wird abgetrennt und die wässrige Phase noch zweimal mit Essigsäureethylester extrahiert. Die vereinigten organischen Phasen werden über Natriumsulfat getrocknet, filtriert und das Lösungsmittel bis zur Trockene entfernt. Das erhaltene Rohprodukt wird durch Flash-Chromatographie an Kieselgel gereinigt (Laufmittel: Cyclohexan/Essigsäureethylester 4:1). Es werden 169 mg (0.37 mmol, 14% d. Th.) eines farblosen Öls erhalten.

LC-MS (Methode 2): Rₜ = 3.02 min; m/z = 459 (M+H)⁺.

### Beispiel 11A

### 4-[(4E)-5-(5-Fluor-2-{[4-(trifluormethyl)benzyl]oxy}phenyl)-3-{2-[4-(methoxycarbonyl)phenyl]-ethyl}pent-4-en-1-yl]benzoesäuremethylester

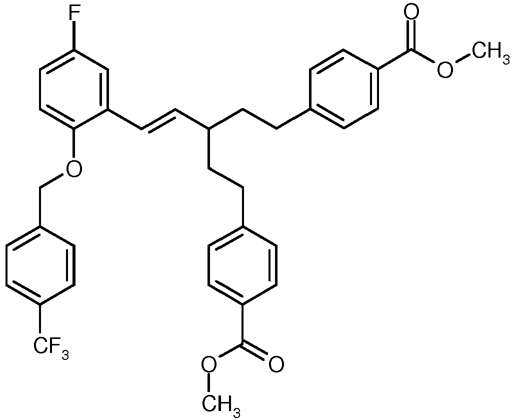

Eine Lösung von 279 mg (0.59 mmol) 4-[(4*E*)-5-(5-Fluor-2-hydroxyphenyl)-3-{2-[4-(methoxycarbonyl)phenyl]ethyl}pent-4-en-1-yl]benzoesäuremethylester in 6.5 ml trockenem Acetonitril wird mit 280 mg (1.2 mmol) 1-(Brommethyl)-4-(trifluormethyl)benzol sowie 162 mg (1.2 mmol) wasserfreiem Kaliumcarbonat versetzt und dann 12 Stunden auf 60°C erhitzt. Anschließend wird der Ansatz filtriert und das Filtrat zur Trockene eingeengt. Der Rückstand wird mittels präparativer HPLC aufgereinigt. Es werden 274 mg (0.43 mmol, 74% d. Th.) eines farblosen Öls isoliert.

¹H-NMR (400 MHz, DMSO-d₆, δ/ppm): 7.81 (4H, d), 7.74-7.62 (4H, m), 7.41-7.34 (1H, m), 7.29 (4H, d), 7.14-7.01 (2H, m), 6.63 (1H, d), 6.27-6.15 (1H, m), 5.24 (2H, s), 3.83 (6H, s), 2.75-2.55 (4H, m), 2.19-2.06 (1H, m), 1.85-1.73 (2H, m), 1.73-1.60 (2H, m).

LC-MS (Methode 7): Rₜ = 3.50 min; m/z = 635 (M+H)⁺.

### Ausführungsbeispiele:

### Beispiel 1

### 4-[(4E)-3-[2-(4-Carboxyphenyl)ethyl]-5-(5-fluor-2-{[4-(trifluormethyl)benzyl]oxy}phenyl)pent-4-en-1-yl]benzoesäure

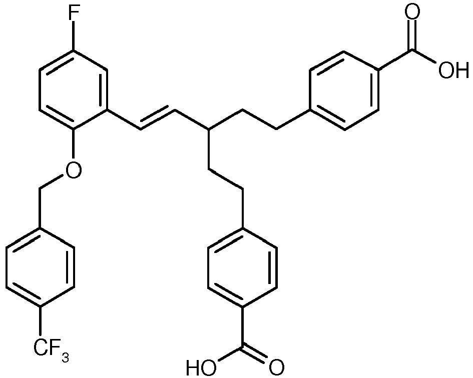

Eine Lösung von 260 mg (0.41 mmol) 4-[(4*E*)-5-(5-Fluor-2-{[4-(trifluormethyl)benzyl]oxy}-phenyl)-3-{2-[4-(methoxycarbonyl)phenyl]ethyl}pent-4-en-1-yl]benzoesäuremethylester in 10 ml THF und 10 ml Wasser wird mit 29 mg (1.23 mmol) Lithiumhydroxid versetzt und 12 Stunden bei 50°C gerührt. Der Ansatz wird danach mit 1 M Salzsäure auf pH 2 gestellt und eingeengt. Der erhaltene Rückstand wird direkt über präparative HPLC gereinigt. Es werden 182 mg (0.29 mmol, 72% d. Th.) der Titelverbindung erhalten.

¹H-NMR (400 MHz, DMSO-d₆, δ/ppm): 12.80 (2H, breit), 7.80 (4H, d), 7.75-7.64 (4H, m), 7.41-7.35 (1H, m), 7.27 (4H, d), 7.15-7.00 (2H, m), 6.66 (1H, d), 6.28-6.18 (1H, m), 5.75 (2H, s), 2.74-2.55 (4H, m), 2.22-2.11 (1H, m), 1.85-1.72 (2H, m), 1.72-1.59 (2H, m).

LC-MS (Methode 9): Rₜ = 4.38 min; m/z = 607 (M+H)⁺.

Die in der folgenden Tabelle aufgeführten Beispiele werden in zur Synthese von Beispiel 1 analoger Weise hergestellt:

| **Beispiel Nr.** | **Beispiel-Struktur *[Edukte]*** | **Analytische Daten** |
|---|---|---|
| **2** | 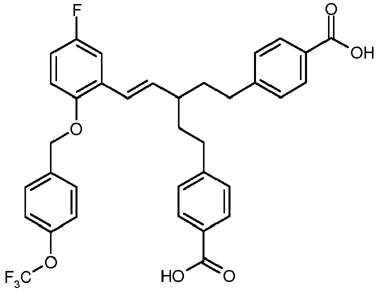 | ¹H-NMR (400 MHz, DMSO-d₆, δ/ppm): 12.76 (2H, breit), 7.82-7.80 (4H, d), 7.60-7.57 (2H, d), 7.39-7.33 (3H, m), 7.28-7.26 (4H, d), 7.12-7.01 (2H, m), 6.65-6.61 (1H, d), 6.25-6.18 (1H, m), 5.17 (2H, s), 2.71-2.50 (4H, m), 2.21-2.11 (1H, m), 1.82-1.74 (2H, m), 1.71-1.61 (2H, m). |
| | *[ausgehend von 1-(Brommethyl)-4-(trifluormethoxy)benzol und Bsp. 9A]* | LC-MS (Methode 8): Rₜ = 4.41 min; m/z = 623 (M+H)⁺. |
| **3** | 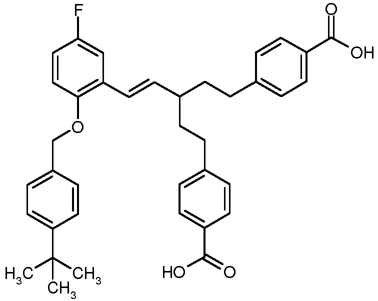 | ¹H-NMR (400 MHz, DMSO-d₆, δ/ppm): 12.77 (2H, breit), 7.83-7.80 (4H, d), 7.40-7.34 (5H, m), 7.29-7.27 (4H, d), 7.14-7.10 (1H, m), 7.06-7.01 (1H, m), 6.67-6.62 (1H, d), 6.24-6.18 (1H, m), 5.09 (2H, s), 2.71-2.49 (4H, m), 2.19-2.10 (1H, m), 1.81-1.73 (2H, m), 1.70-1.63 (2H, m), 1.24 (9H, s). |
| | *[ausgehend von 4-(tert.-Butyl)benzylbromid und Bsp. 9A]* | LC-MS (Methode 8): Rt = 4.51 min; m/z = 593 (M-H)⁻. |
| **4** | 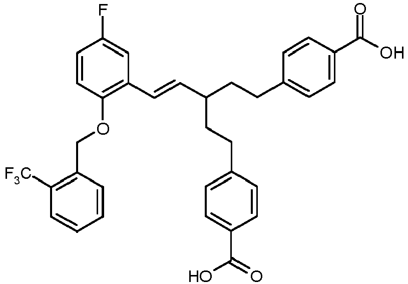 | ¹H-NMR (400 MHz, DMSO-d₆, δ/ppm): 12.76 (2H, breit), 7.82-7.75 (6H, m), 7.71-7.67 (1H, t), 7.60-7.56 (1H, t), 7.40-7.37 (1H, m), 7.24-7.22 (4H, d), 7.13-7.03 (2H, m), 6.60-6.56 (1H, d), 6.22-6.15 (1H, m), 5.25 (2H, s), 2.71-2.50 (4H, m), 2.10-2.08 (1H, m), 1.75-1.73 (2H, m), 1.66-1.59 (2H, m). |
| | *[ausgehend von 1-(Brommethyl)-2-(trifluormethyl)benzol und Bsp. 9A]* | LC-MS (Methode 8): Rt = 4.28 min; m/z = 607 (M+H)⁺. |
| **5** | 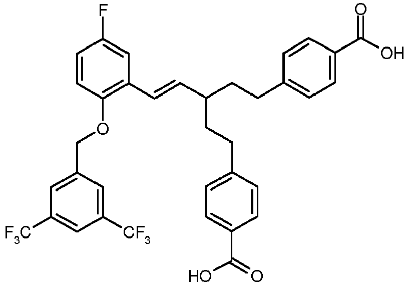 | ¹H-NMR (400 MHz, DMSO-d₆, δ/ppm): 12.75 (2H, breit), 8.16 (2H, s), 8.06 (1H, s), 7.77-7.75 (4H, d), 7.40-7.37 (1H, m), 7.24-7.22 (4H, d), 7.15-7.05 (2H, m), 6.65-6.61 (1H, d), 6.26-6.20 (1H, m), 5.35 (2H, s), 2.68-2.49 (4H, m), 2.14-2.12 (1H, m), 1.78-1.76 (2H, m), 1.66-1.61 (2H, m). |
| | *[ausgehen von 1-(Brommethyl)-3,5-bis(trifluormethyl)benzol und Bsp. 9A]* | LC-MS (Methode 8): Rₜ = 4.34 min; m/z = 675 (M+H)⁺. |
| **6** | 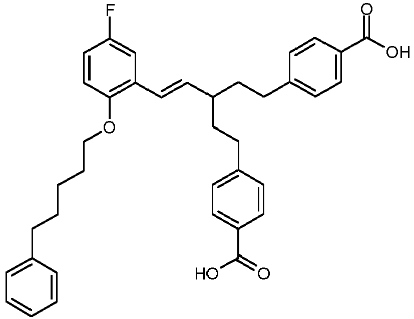 | ¹H-NMR (400 MHz, DMSO-d₆, δ/ppm): 12.78 (2H, breit), 7.85-7.83 (4H, d), 7.34-7.29 (5H, m), 7.22-7.18 (2H, t), 7.13-7.09 (3H, m), 7.03-6.98 (2H, m), 6.60-6.55 (1H, d), 6.22-6.15 (1H, m), 3.96 (2H, t), 2.71-2.50 (6H, m), 2.14-2.12 (1H, m), 1.78-1.75 (4H, m), 1.70-1.57 (4H, m), 1.48-1.43 (2H, m). |
| | *[ausgehend von Bsp. 1A und Bsp. 9A]* | LC-MS (Methode 8): Rt = 4.54 min; m/z = 595 (M+H)⁺. |
| **7** | 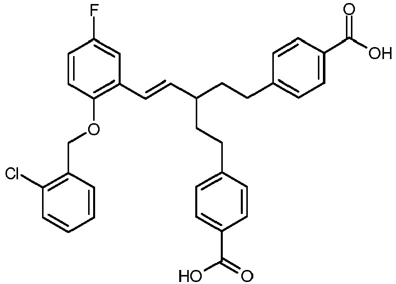 | ¹H-NMR (400 MHz, DMSO-d₆, δ/ppm): 12.77 (2H, breit), 7.80-7.78 (4H, d), 7.62-7.60 (1H, m), 7.51-7.49 (1H, m), 7.40-7.33 (3H, m), 7.26-7.24 (4H, d), 7.16-7.13 (1H, m), 7.08-7.03 (1H, m), 6.63-6.59 (1H, d), 6.23-6.17 (1H, m), 5.18 (2H, s), 2.67-2.50 (4H, m), 2.12-2.10 (1H, m), 1.76-1.75 (2H, m), 1.67-1.61 (2H, m). |
| | *[ausgehend von 2-Chlorbenzylbromidund Bsp. 9A]* | LC-MS (Methode 8): Rₜ = 4.28 min; m/z = 571 (M-H)⁻. |

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro:

Kaninchen werden durch intravenöse Injektion von Thiopental-Natrium narkotisiert bzw. getötet (ca. 50 mg/kg) und entblutet. Die Arteria Saphena wird entnommen und in 3 mm breite Ringe geteilt. Die Ringe werden einzeln auf je einem triangelförmigen, am Ende offenen Häkchenpaar aus 0.3 mm starkem Spezialdraht (Remanium^{®}) montiert. Jeder Ring wird unter Vorspannung in 5 ml-Organbäder mit 37°C warmer, carbogenbegaster Krebs-Henseleit-Lösung folgender Zusammensetzung gebracht: NaCl 119 mM; KCl 4.8 mM; CaCl₂ x 2 H₂O 1 mM; MgSO₄ x 7 H₂O 1.4 mM; KH₂PO₄ 1.2 mM; NaHCO₃ 25 mM; Glucose 10 mM; Rinderserumalbumin 0.001%. Die Kontraktionskraft wird mit Statham UC2-Zellen erfasst, verstärkt und über A/D-Wandler (DAS-1802 HC, Keithley Instruments, München) digitalisiert sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50% zu reduzieren (IC₅₀-Wert). Das Standard-Applikationsvolumen beträgt 5 µl. Der DMSO-Anteil in der Badlösung entspricht 0.1%.

Ein repräsentatives Ergebnis zu den erfindungsgemäßen Verbindungen ist in Tabelle 1 aufgeführt:

**Tabelle 1: Gefäßrelaxierende Wirkung in vitro**

| **Beispiel Nr.** | **IC₅₀ [nM]** |
|---|---|
| 1 | 250 |

### B-2. Stimulation der rekombinanten löslichen Guanylatcyclase (sGC) in vitro:

Die Untersuchungen zur Stimulation der rekombinanten löslichen Guanylatcyclase (sGC) durch die erfindungsgemäßen Verbindungen mit und ohne Natriumnitroprussid sowie mit und ohne den Häm-abhängigen sGC-Inhibitor 1*H*-1,2,4-Oxadiazol-(4,3a)-chinoxalin-1-on (ODQ) werden nach der in folgender Literaturstelle im Detail beschriebenen Methode durchgeführt: M. Hoenicka, E.M.

Becker, H. Apeler, T. Sirichoke, H. Schroeder, R. Gerzer und J.-P. Stasch, "Purified soluble guanylyl cyclase expressed in a baculovirus/Sf9 system: Stimulation by YC-1, nitric oxide, and carbon oxide", J. Mol. Med. 77 (1999), 14-23. Die Häm-freie Guanylatcyclase wird durch Zugabe von Tween 20 zum Probenpuffer (0.5% in der Endkonzentration) erhalten.

Die Aktivierung der sGC durch eine Prüfsubstanz wird als n-fache Stimulation der Basalaktivität angegeben. Das Ergebnis für Beispiel 1 ist in Tabelle 2 gezeigt:

**Tabelle 2: Stimulation (n-fach) der rekombinanten löslichen Guanylatcyclase (sGC) in vitro durch Beispiel 1**

| **Konzentration Beispiel 1 [µM]** | **Häm-haltige sGC** | | | **Häm-freie sGC Basal** |
|---|---|---|---|---|
| | **Basal** | **+ 0.1 µM DEA/NO** | **+ 10 µM ODQ** | |
| 0.0 | 1.0 | 25.0 | 5.3 | 1.0 |
| 0.01 | 1.3 | 24.1 | 7.0 | 1.0 |
| 0.1 | 1.9 | 25.5 | 16.2 | 2.3 |
| 1 | 6.4 | 33.4 | 60.6 | 9.1 |
| 10 | 11.0 | 41.5 | 92.2 | 16.7 |

[DEA/NO = 2-(N,N-Diethylamino)diazenolat-2-oxid; ODQ = 1*H*-1,2,4-Oxadiazol-(4,3a)-chinoxalin-1-on].

Aus Tabelle 2 ist ersichtlich, dass eine Stimulation sowohl des Häm-haltigen als auch des Hämfreien Enzyms erreicht wird. Weiterhin zeigt die Kombination aus Beispiel 1 und 2-(*N,N-*Diethyl-amino)diazenolat-2-oxid (DEA/NO), einem NO-Donor, keinen synergistischen Effekt, d.h. die Wirkung von DEA/NO wird nicht potenziert, wie dies bei einem über einen Häm-abhängigen Mechanismus wirkenden sGC-Aktivator zu erwarten wäre. Darüber hinaus wird die Wirkung des erfindungsgemäßen sGC-Aktivators durch den Häm-abhängigen Inhibitor der löslichen Guanylatcyclase ODQ nicht blockiert, sondern sogar gesteigert. Die Ergebnisse aus Tabelle 2 belegen somit den Wirkmechanismus der erfindungsgemäßen Verbindungen als Aktivatoren der löslichen Guanylatcyclase.

### B-3. Radiotelemetrische Messung von Blutdruck und Herzfrequenz an wachen SH-Ratten

Für die im Folgenden beschriebenen Messungen an wachen SH-Ratten wird ein im Handel erhältliches Telemetriesystem der Firma Data Sciences International DSI, USA, eingesetzt.

Das System besteht aus 3 Hauptkomponenten: (*1*) Implantierbare Sender, (*2*) Empfänger, die über einen Multiplexer mit einem (*3*) Datenakquisitionscomputer verbunden sind. Die Telemetrieanlage ermöglicht eine kontinuierliche Erfassung von Blutdruck und Herzfrequenz an wachen Tieren in ihrem gewohnten Lebensraum.

Die Untersuchungen werden an ausgewachsenen weiblichen, spontan-hypertensiven Ratten (SH-Ratten) mit einem Körpergewicht von >200 g durchgeführt. Die Versuchstiere werden nach Senderimplantation einzeln in Makrolon-Käfigen Typ 3 gehalten. Sie haben freien Zugang zu Standardfutter und Wasser. Der Tag/Nacht-Rhythmus im Versuchslabor wird per Raumbeleuchtung um 6:00 Uhr morgens und um 19:00 Uhr abends gewechselt.

Die eingesetzten Telemetriesender (TAM PA-C40, DSI) werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobarbital (Nembutal, Sanofi, 50 mg/kg i.p.) narkotisiert und an der Bauchseite weiträumig rasiert und desinfiziert. Nach Eröffnung des Bauchraumes entlang der Linea alba wird der flüssigkeitsgefüllte Messkatheter des Systems oberhalb der Bifurcation nach cranial in die Aorta descendens eingesetzt und mit Gewebekleber (VetBonD™, 3M) befestigt. Das Sendergehäuse wird intraperitoneal an der Bauchwandmuskulatur fixiert und die Wunde schichtweise verschlossen. Postoperativ wird zur Infektionsprophylaxe ein Antibiotikum verabreicht (Tardomyocel COMP, Bayer, 1 ml/kg s.c.).

### Versuchsablauf:

Die zu untersuchenden Substanzen werden jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert. Eine Lösungsmittel-behandelte Gruppe von Tieren wird als Kontrolle eingesetzt.

Die Telemetrie-Messeinrichtung ist für 24 Tiere konfiguriert. Jeder Versuch wird unter einer Versuchsnummer registriert.

Den in der Anlage lebenden instrumentierten Ratten ist jeweils eine eigene Empfangsantenne zugeordnet (1010 Receiver, DSI). Die implantierten Sender sind über einen eingebauten Magnetschalter von außen aktivierbar und werden bei Versuchsvorlauf auf Sendung geschaltet. Die ausgestrahlten Signale können durch ein Datenakquisitionssystem (Dataquest™ A.R.T. for Windows, DSI) online erfasst und entsprechend aufgearbeitet werden. Die Ablage der Daten erfolgt jeweils in einem hierfür eröffneten Ordner, der die Versuchsnummer trägt.

Im Standardablauf werden über je 10 Sekunden Dauer gemessen: (1) systolischer Blutdruck (SBP), (2) diastolischer Blutdruck (DBP), (3) arterieller Mitteldruck (MAP) und (4) Herzfrequenz (HR).

Die Messwerterfassung wird rechnergesteuert in 5 Minuten-Abständen wiederholt. Die als Absolutwert erhobenen Quelldaten werden im Diagramm mit dem aktuell gemessenen Barometerdruck korrigiert und in Einzeldaten abgelegt. Weitere technische Details sind in der Dokumentation der Herstellerfirma (DSI) aufgeführt.

Die Verabreichung der Prüfsubstanzen erfolgt am Versuchstag um 9:00 Uhr. Im Anschluss an die Applikation werden die oben beschriebenen Parameter über 24 Stunden gemessen. Nach Versuchsende werden die erhobenen Einzeldaten mit der Analysis-Software (Dataquest™ A.R.T. Analysis) sortiert. Als Leerwert wird der Zeitpunkt 2 Stunden vor Substanz-Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten-Mittelwert, 30 Minuten-Mittelwert) und als Textdatei auf einen Datenträger übertragen. Die so vorsortierten und komprimierten Messwerte werden in Excel-Vorlagen übertragen und tabellarisch dargestellt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I-A) 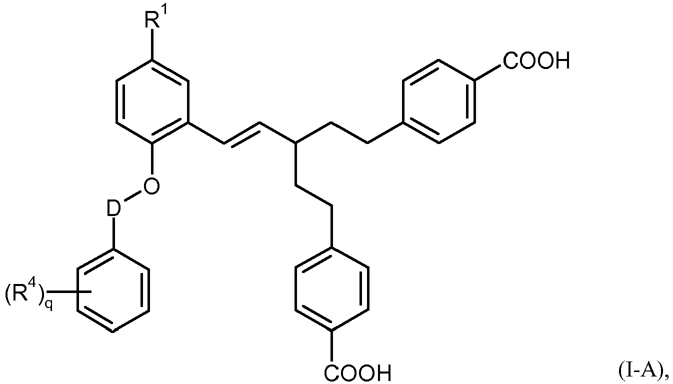 in welcher
D für (C₁-C₇)-Alkandiyl steht,
R¹ für Wasserstoff oder Fluor steht,
R⁴ für einen Substituenten ausgewählt aus der Reihe Fluor, Chlor, Methyl, *tert*.-Butyl, Trifluormethyl, Methoxy und Trifluormethoxy steht,
und
q für die Zahl 0, 1 oder 2 steht, wobei für den Fall, dass der Substituent R⁴ zweifach auftritt, seine Bedeutungen gleich oder verschieden sein können,
sowie ihre Salze, Solvate und Solvate der Salze.

2. Verfahren zur Herstellung einer Verbindung der Formel (I-A), wie in Anspruch 1 definiert, **dadurch gekennzeichnet, dass** man Verbindungen der Formel (II) 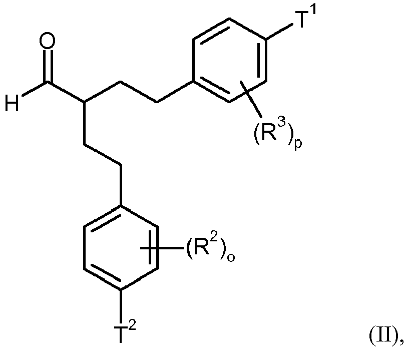
in welcher R², R³, o und p jeweils die in Anspruch 1 angegebenen Bedeutungen haben und
T¹ und T² gleich oder verschieden sind und für (C₁-C₄)-Alkoxycarbonyl stehen, entweder
[A] in einem inerten Lösungsmittel in Gegenwart einer Base mit einer Verbindung der Formel (III-A) 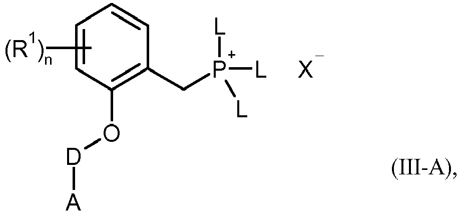 in welcher A, D, R¹ und n jeweils die inAnspruch 1 angegebenen Bedeutungen haben und
L für Phenyl oder o-, m- oder p-Tolyl
und
X für Halogenid oder Tosylat steht,
zu Verbindungen der Formel (IV-A) 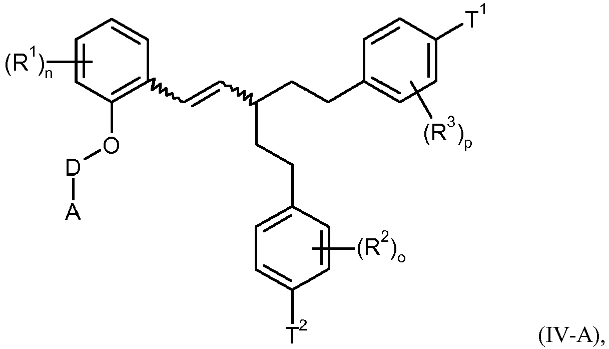 in welcher A, D, R¹, R², R³, n, o, p, T¹ und T² jeweils die oben angegebenen Bedeutungen haben,
umsetzt
oder
[B] in einem inerten Lösungsmittel in Gegenwart einer Base mit einer Verbindung der Formel (III-B) 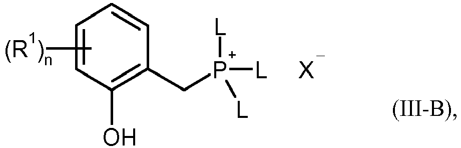 *in* welcher R¹, n, L und X jeweils die oben angegebenen Bedeutungen haben,
zunächst zu Verbindungen der Formel (IV-B) 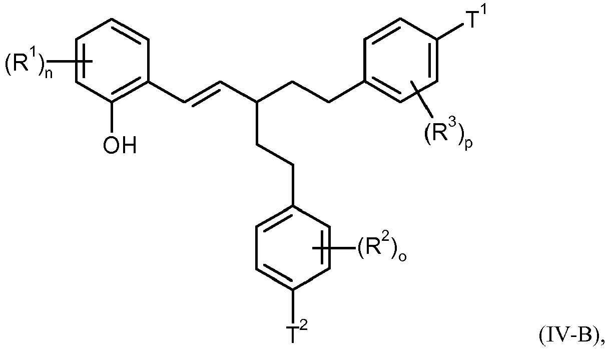
*in* welcher R¹, R², R³, n, o, p, T¹ und T² jeweils die oben angegebenen Bedeutungen haben,
umsetzt und diese anschließend in einem inerten Lösungsmittel in Gegenwart einer Base mit einer Verbindung der Formel (V)
A-D-Q (V),
in welcher A und D die in den Anspruch 1 angegebenen Bedeutungen haben und
Q für eine Abgangsgruppe, wie beispielsweise Halogen, Tosylat oder Mesylat, steht,
zu Verbindungen der Formel (IV-C) 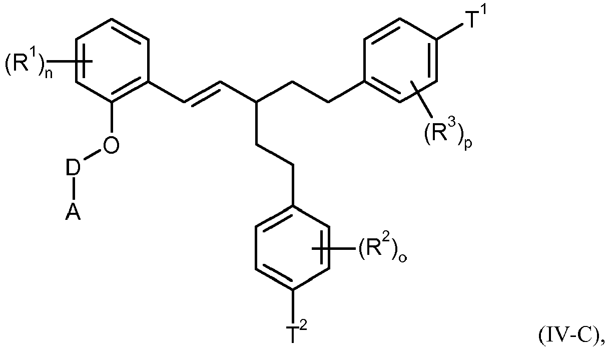 *in* welcher A, D, R¹, R², R³, n, o, p, T¹ und T² jeweils die oben angegebenen Bedeutungen haben,
kuppelt
und die resultierenden Verbindungen der Formel (IV-A) bzw. (IV-C) dann durch Hydrolyse der Ester-Gruppen T¹ und T² in die Dicarbonsäuren der Formel (I) überführt
und die Verbindungen der Formel (I-A) gegebenenfalls nach dem Fachmann bekannten Methoden in ihre Enantiomere und/oder Diastereomere trennt und/oder gegebenenfalls mit den entsprechenden (i) Lösungsmitteln und/oder (ii) Basen oder Säuren zu ihren Solvaten, Salzen und/oder Solvaten der Salze umsetzt.

3. Verbindung, wie in Anspruch 1 definiert, zur Behandlung und/oder Prävention von Krankheiten.

4. Verwendung einer Verbindung, wie in Anspruch 1 definiert, zur Herstellung eines Arzneimittels zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

5. Arzneimittel enthaltend eine Verbindung, wie in Anspruch 1 definiert, in Kombination mit einem inerten, nicht-toxischen, pharmazeutisch geeigneten Hilfsstoff.

6. Arzneimittel enthaltend eine Verbindung, wie in Anspruch 1 definiert, in Kombination mit einem oder mehreren weiteren Wirkstoffen ausgewählt aus der Gruppe bestehend aus organischen Nitraten, NO-Donatoren, cGMP-PDE-Inhibitoren, Stimulatoren der Guanylatcyclase, antithrombotisch wirkenden Mitteln, den Blutdruck senkenden Mitteln sowie den Fettstoffwechsel verändernden Mitteln.

7. Arzneimittel nach Anspruch 5 oder 6 zur Behandlung und/oder Prävention von Herzinsuffizienz, Angina pectoris, Hypertonie, pulmonaler Hypertonie, Ischämien, Gefäßerkrankungen, thromboembolischen Erkrankungen und Arteriosklerose.

## Claims

1. Compound of the formula (I-A) 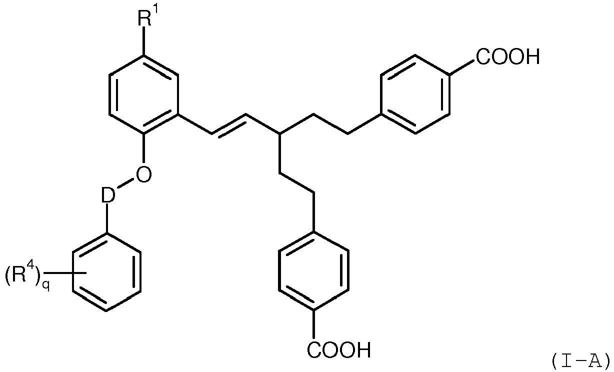 in which
D represents (C₁-C₇)-alkanediyl,
R¹ represents hydrogen or fluorine,
R⁴ represents a substituent selected from the group consisting of fluorine, chlorine, methyl, *tert*-butyl, trifluoromethyl, methoxy and trifluoromethoxy,
and
q represents the number 0, 1 or 2,
where, if the substituent R⁴ is present twice, its meanings may be identical or different,
and the salts, solvates and solvates of the salts thereof.

2. Process for preparing a compound of the formula (I-A) as defined in Claim 1, **characterized in that** compounds of the formula (II) 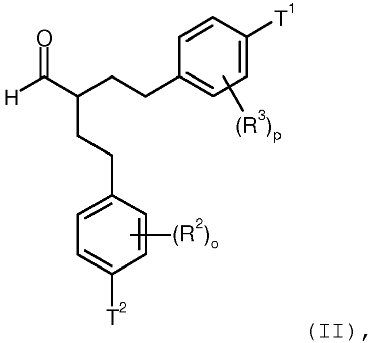
in which R², R³, o and p each have the meanings given in Claim 1 and
T¹ and T² are identical or different and represent (C₁-C₄)-alkoxycarbonyl,
are either
[A] converted in an inert solvent in the presence of a base with a compound of the formula (III-A) 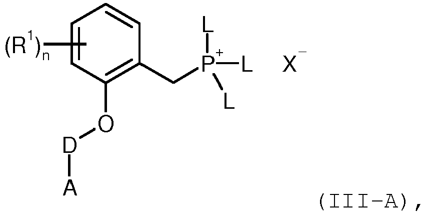 in which A, D, R¹ and n each have the meanings given in Claim 1 and
L represents phenyl or o-, m- or p-tolyl
and
X represents halide or tosylate,
into compounds of the formula (IV-A) 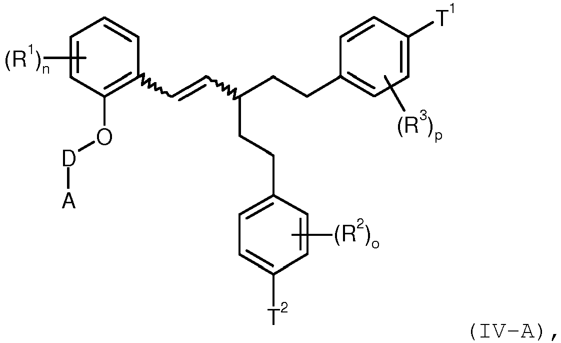 in which A, D, R¹, R², R³, n, o, p, T¹ and T² each have the meanings given above,
or
[B] converted in an inert solvent in the presence of a base with a compound of the formula (III-B) 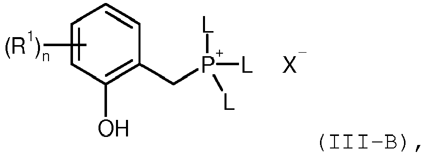 in which R¹, n, L and X each have the meanings given above,
initially into compounds of the formula (IV-B) 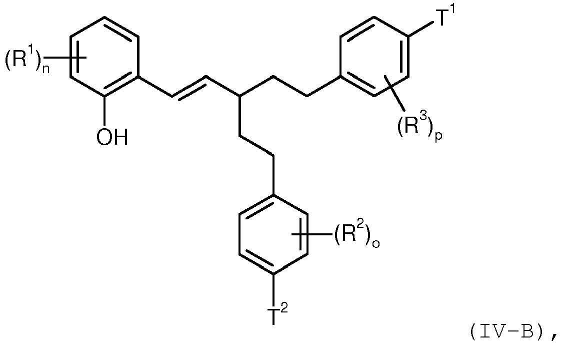
in which R¹, R², R³, n, o, p, T¹ and T² each have the meanings given above,
and these are then coupled in an inert solvent in the presence of a base with a compound of the formula (V)
A-D-Q (V),
in which A and D have the meanings given in Claim 1 and
Q represents a leaving group, such as, for example, halogen, tosylate or mesylate, to give compounds of the formula (IV-C) 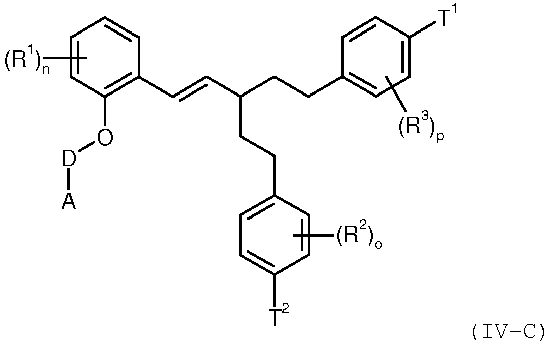
in which A, D, R¹, R², R³, n, o, p, T¹ and T² each have the meanings given above,
and the resulting compounds of the formula (IV-A) or (IV-C) are then converted by hydrolysis of the ester groups T¹ and T² into the dicarboxylic acids of the formula (I),
and the compounds of the formula (I-A) are separated where appropriate by methods known to the skilled person into their enantiomers and/or diastereomers, and/or where appropriate reacted with the appropriate (i) solvents and/or (ii) bases or acids to give the solvates, salts and/or solvates of the salts thereof.

3. Compound as defined in Claim 1 for the treatment and/or prevention of diseases.

4. Use of a compound as defined in Claim 1 for producing a medicament for the treatment and/or prevention of heart failure, angina pectoris, hypertension, pulmonary hypertension, ischemias, vascular disorders, thromboembolic disorders and arteriosclerosis.

5. Medicament comprising a compound as defined in Claim 1 in combination with an inert, non-toxic, pharmaceutically suitable excipient.

6. Medicament comprising a compound as defined in Claim 1 in combination with one or more further active ingredients selected from the group consisting of organic nitrates, NO donors, cGMP-PDE inhibitors, stimulators of guanylate cyclase, agents having antithrombotic activity, agents lowering blood pressure, and agents altering lipid metabolism.

7. Medicament according to Claim 5 or 6 for the treatment and/or prevention of heart failure, angina pectoris, hypertension, pulmonary hypertension, ischemias, vascular disorders, thromboembolic disorders and arteriosclerosis.

## Revendications

1. Composé de formule (I-A) 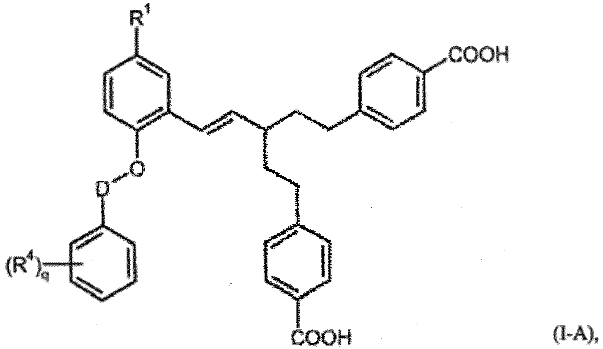 dans laquelle
D représente (C₁-C₇)-alcanediyle,
R¹ représente hydrogène ou fluor
R⁴ représente un substituant choisi dans la série fluor, chlore, méthyle, *tert*-butyle, trifluorométhyle, méthoxy et trifluorométhoxy,
et
q représente le nombre 0, 1 ou 2 où, dans le cas où le substituant R⁴ apparaît deux fois, ses significations peuvent être identiques ou différentes
ainsi que ses sels, solvates et solvates des sels.

2. Procédé pour la préparation d'un composé de formule (I-A), telle que définie dans la revendication 1, **caractérisé en ce qu'**on transforme des composés de formule (II) 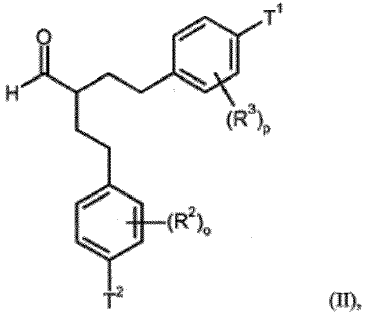 dans laquelle R², R³, o et p présentent à chaque fois les significations indiquées dans la revendication 1 et
T¹ et T² sont identiques ou différents et représentent (C₁-C₄)-alcoxycarbonyle,
soit
[A] dans un solvant inerte en présence d'une base, avec un composé de formule (III-A) 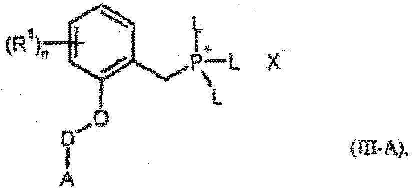 dans laquelle A, D, R¹ et n présentent à chaque fois les significations indiquées dans la revendication 1 et
L représente phényle ou o-toluyle, mtoluyle ou p-toluyle
et
X représente halogénure ou tosylate,
en composés de formule (IV-A) 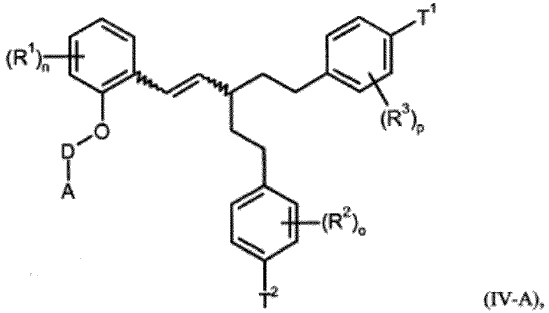 dans laquelle A, D, R¹, R², R³, n, o, p, T¹ et T² présentent à chaque fois les significations indiquées ci-dessus,
ou
[B] dans un solvant inerte en présence d'une base, avec un composé de formule (III-B) 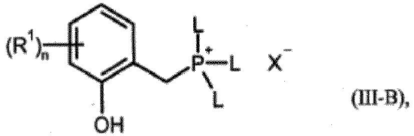 dans laquelle R¹, n, L et X présentent à chaque fois les significations indiquées ci-dessus,
d'abord en composés de formule (IV-B) 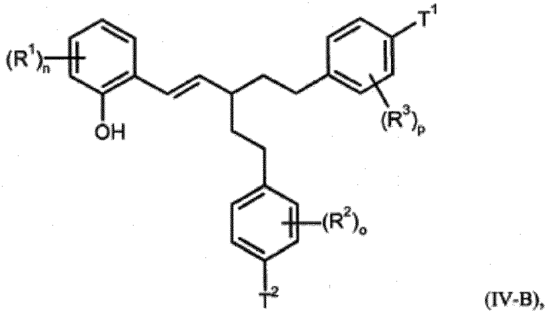
dans laquelle R¹, R², R³, n, o, p, T¹ et T² présentent à chaque fois les significations indiquées ci-dessus,
et on couple ensuite ceux-ci, dans un solvant inerte en présence d'une base, avec un composé de formule (V)
A-D-Q (V)
dans laquelle A et D présentent les significations indiquées dans la revendication 1 et
Q représente un groupe partant, tel que par exemple halogène, tosylate ou mésylate,
en composés de formule (IV-C) 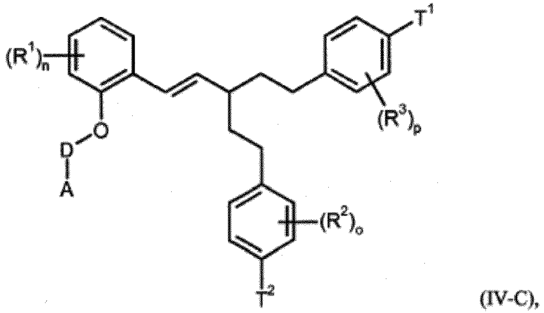 dans laquelle A, D, R¹, R², R³, n, o, p, T¹ et T² présentent à chaque fois les significations indiquées ci-dessus,
et on transforme ensuite les composés résultants des formules (IV-A) ou (IV-C) par hydrolyse des groupes ester T¹ et T² en acides dicarboxyliques de formule (I)
et on sépare les composés de formule (I-A) le cas échéant selon des procédés connus par l'homme du métier en leurs énantiomères et/ou diastéréo-isomères et/ou on les transforme le cas échéant avec (i) les solvants et/ou (ii) les bases ou les acides correspondants en leur solvates, sels et/ou solvates des sels.

3. Composé, tel que défini dans la revendication 1, pour le traitement et/ou la prévention de maladies.

4. Utilisation d'un composé, tel que défini dans la revendication 1, pour la préparation d'un médicament pour le traitement et/ou la prévention de l'insuffisance cardiaque, de l'angine de poitrine, de l'hypertonie, de l'hypertonie pulmonaire, d'ischémies, de maladies vasculaires, de maladies thromboemboliques et de l'artériosclérose.

5. Médicament contenant un composé tel que défini dans la revendication 1 en combinaison avec un adjuvant inerte, non toxique, pharmaceutiquement approprié.

6. Médicament contenant un composé tel que défini dans la revendication 1 en combinaison avec une ou plusieurs autres substances actives, choisies dans le groupe constitué par les nitrates organiques, les donneurs de NO, les inhibiteurs de cGMP-PDE, les stimulateurs de la guanylate-cyclase, les agents à effet antithrombotique, les agents abaissant la tension ainsi que les agents modifiant le métabolisme des graisses.

7. Médicament selon la revendication 5 ou 6 pour le traitement et/ou la prévention de l'insuffisance cardiaque, de l'angine de poitrine, de l'hypertonie, de l'hypertonie pulmonaire, d'ischémies, de maladies vasculaires, de maladies thromboemboliques et de l'artériosclérose.
